# EUROPEAN PATENT APPLICATION

(11) **EP 4 260 825 A1**
(43) Date of publication of application: **18.10.2023**
(21) Application number: 23167464.9
(22) Date of filing: 12.04.2023
(51) Int. Cl.: A61B 17/74

(54) **SCREWLESS LATERAL ANCHORING**

(30) Priority: 12.04.2022 US 202263329944 P
(71) Applicant: Stryker European Operations Limited, Carrigtwohill, Co. Cork T45 HX08 (IE)
(72) Inventor: ZANDER, Nils, 24340 ECKERNFÖRDE (DE)
(74) Representative: Regimbeau

(57) **Abstract**

A fracture fixation system includes a barrel extending along a barrel axis from a proximal end to a distal end having a peripheral wall. The barrel defines a passage through the barrel that extends along the barrel axis. The peripheral wall defines a transverse bore passing through opposing sides of the peripheral wall at the proximal end of the barrel in a direction that is substantially transverse to the barrel axis. The fixation system includes a peg extending along a peg axis and configured for insertion into the passage. The fixation system includes a flange configured for insertion into the transverse bore of the peripheral wall.

## Description

### CROSS-REFERENCE TO RELATED APPLICATIONS

The present application claims the benefit of the priority of U.S. Provisional Application No. 63/329,944 filed on April 12, 2022, the disclosure of which is hereby incorporated by reference herein in its entirety.

### BACKGROUND OF THE INVENTION

The present invention relates to orthopedic surgical devices used to join and promote healing of fractured bone, and more particularly, and not by limitation, devices used to fixate proximal femoral fractures.

The surgical treatment of femoral neck fractures utilizing internal fixation remains challenging, especially for dislocated unstable fractures. There are a variety of devices used to treat fractures of the femur, humerus, tibia, and other long bones. For example, fractures of the femoral neck, head, and intertrochanteric region have been successfully treated with a variety of internal fixation means such as compression screw assemblies. Compression hip and bone screw devices for use in fixating a fractured bone during the healing process have been used for years. It is mainstream practice for surgeons to utilize cannulated compression screws (CCS) or sliding hip screws (SHS) as compression screws in internal fixation systems.

For many surgeons and customers, the utilization of CCS or SHS devices remain the treatment of choice. However, CCS accounts for up to nearly 30% of hip fracture failures and their disadvantages are well documented. In particular, CCS devices are not angularly stable, have insufficient rotation control, and suffer from uncontrolled shortening of the femoral neck and limited resistance against shear forces. The disadvantages of SHS are that an additional anti-rotation screw is required with limited space particularly in small anatomies, that they have large lateral footprints, and also that they create a potential collision with a retrograde nail in the case of ipsilateral neck-shaft fixation.

Additionally, problems may result from weakened or poor-quality bone that is adjacent to the fracture site. Often times the bone adjacent to the fracture is weak and is prone to damage when exposed to compression. For example, there could be uncontrolled shortening of the femoral head when the femoral head compresses towards or into the fracture site. In extreme cases, uncontrolled shortening may cause the femoral head to be compressed all the way into the trochanteric region of the femur.

Thus, it would be desirable to provide a fracture fixation system to improve on the prior art disadvantages.

### BRIEF SUMMARY OF THE INVENTION

Described herein is a fracture fixation device and system used particularly to mount and install on a femur to provide support the femur and assist with healing of a proximal femoral fracture. The fixation device may include a barrel defining a passageway and adapted to receive a peg. The peg is sized and shaped to fit within the passage way of the barrel and extend further distally through a distal opening in the barrel. The peg may be inserted into the passageway of the barrel without passing completely through the barrel, such that a proximal end of the peg is held or disposed within a portion of the barrel. In some examples, the peg and barrel may be two separate pieces, and in other examples, the peg and barrel may be monolithic. The peg and barrel may each have corresponding figure-8 shapes, wherein each of the peg and barrel define an upper cylindrical portion and a lower cylindrical portion.

The barrel further defines an additional passageway or bore extending in a different direction than the passageway that receives the peg. The second passageway is generally transverse to the first passageway, such that it extends across the barrel through each of the first and second cylindrical portions, and is located near the proximal end of the barrel. The second passageway is adapted to receive a flange or latch that may be inserted into the barrel after the barrel is implanted in the femur. The flange may be inserted through the upper cylindrical portion of the barrel and pushed down in the distal or inferior direction, such that at least a portion of the flange protrudes from the lower cylindrical portion of the barrel and extends into the femur, particularly the intramedullary canal. The flange is helpful to further secure the fixation device within the femur.

In some examples, the fixation device will include a screw installed therein to lock the flange after it has been pushed into the desired securing position. That is, the flange may include an opening sized to receive the internal screw of the device, so that after the flange is actuated into the secure position, the screw may be accessed by the operator and actuated to be passed through the opening of the flange, thereby locking the flange in the securing position. In other examples, the fixation system may include an additional external screw that may be inserted directly into the femur to pass through the flange after the flange has been actuated into the securing position to lock the flange in the securing position. In some examples, the fixation device comprising the barrel, peg, flange, and optionally the internal screw may be pre-assembled prior to being implanted in the femur.

In one aspect of the disclosure, a fracture fixation system includes a barrel extending along a barrel axis from a proximal end to a distal end and having a wall, the barrel defining a passage through the barrel that extends along the barrel axis, wherein the wall defines a transverse bore passing through opposing sides of the wall at the proximal end of the barrel in a direction that is substantially transverse to the barrel axis. The fracture fixation system further includes a peg extending along a peg axis and configured for insertion into the passage and a flange configured for insertion into the transverse bore of the wall.

Further to the fracture fixation system according to the first aspect of the disclosure, the flange may be moveable in opposing directions through the transverse bore of the barrel. In a pre-implanted configuration, the flange may extend from one of the opposing sides of the walls. In the pre-implanted configuration, the flange may extend in a superior direction from the barrel. In an implanted configuration, the flange may extend through both of the opposing sides of the walls. In the implanted configuration, a proximal end of the flange may be flush with the barrel. In the implanted configuration with the flange in a latched configuration, the flange may protrude in a superior direction from the barrel. In the implanted configuration, a distal portion of the flange may abut an inner lateral cortex of a femur. The system may include a screw configured to be accessible through an oblong opening defined in the flange. The barrel may include a superior cylindrical portion defining a superior portion of the transverse bore and the barrel includes an inferior cylindrical portion defining an inferior portion of the transverse bore. The inferior portion of the transvers bore may have a diameter larger than a diameter of the superior portion of the transverse bore. An inner surface of the wall of the barrel may have a non-circular shape in a plane perpendicular to the barrel axis, and a body of the peg may have an outer surface defining a non-circular shape in a plane perpendicular to the peg axis. The non-circular shapes of the inner surface of the wall of the barrel and the outer surface of the body of the peg may be figure-8 shapes. The peg may be disposed within the passage and the flange may be disposed within the transverse bore of the wall. The flange may block the proximal movement of the peg out of the passage. The flange may include a superior stop configured to prevent movement of the flange out of the transverse bore in an inferior direction. The flange may include a distal stop configured to prevent movement of the flange out of the transverse bore in a superior direction. The wall may be a peripheral wall. The fracture fixation system of the first aspect of the disclosure may be provided in a kit along with a screw for insertion through a distal portion of the flange.

According to a second aspect of the disclosure, a method of using a fracture fixation system includes drilling a bore hole in a bone; mounting the fracture fixation system to the bone, including inserting a barrel into the bore hole, the barrel including a wall extending along a barrel axis and a peg coupled to the barrel, the barrel defining a transverse bore passing through both sides of the wall at a proximal end of the barrel; and moving a flange extending through the proximal end of the barrel relative to the barrel.

Further to the method of the second aspect of the disclosure, the flange may be moved through the transverse bore in the proximal end of the barrel at an angle that is substantially transverse to the barrel axis. The bone may be a femur and moving the flange may include passing the flange into an intramedullary canal of the femur. When the wall of the barrel is inserted into the bore hole, the flange may be in a first position. After the wall of the barrel is inserted into the bore hole, the step of moving the flange may include moving the flange in an inferior direction toward a second position. When the flange is moved to the second position, the flange may extend into the intramedullary canal. The method may further include actuating a screw through an opening defined by the flange. The method may further include adjusting the flange within the transverse bore in a proximal-distal direction. The method may further include rotating an internal screw coupled to the flange to press the flange toward a lateral cortex of the femur. Drilling the bore hole may include drilling a superior bore through a femoral neck and into a femoral head of a femur. Drilling the bore hole may include drilling an inferior bore through the femoral neck and into the femoral head to at least partially overlap the superior bore to create a bore hole having a figure-8 shape. The wall may have a figure-8 shape in a plane perpendicular to the barrel axis, and the step of mounting the fracture fixation system may include inserting a peg into a passage defined through the barrel such that a distal end of the peg extends into communication with the femoral head, a body of the peg having an outer surface defining a figure-8 shape in a plane perpendicular to a peg axis along which the peg extends. The bone may be a femur and the bore hole may be drilled in a trochanter of the femur.

According to a third aspect of the disclosure, a fracture fixation system includes an elongate body extending along a body axis from a proximal end to a distal end, the elongate body defining a passage through the body that extends along the body axis, and the elongate body defining a transverse bore passing through a proximal end of the elongate body in a direction that is substantially transverse to the body axis, wherein the elongate body is configured to be inserted into a neck of a femur. The fracture fixation system further includes a flange configured for insertion into the transverse bore of the elongate body, wherein the latch is configured to be inserted into an intramedullary canal of the femur. The elongate body may be a barrel.

### BRIEF DESCRIPTION OF THE DRAWINGS

The drawings described herein are for illustrative purposes only of the selected embodiments and are not all possible implementations and thus are not intended to limit the scope of the present disclosure.
FIG. 1 is a schematic view of a fracture fixation system according to an embodiment of the disclosure implanted into a femur in an unlatched configuration.
FIG. 2 is a schematic view of the fracture fixation system of FIG. 1 implanted into the femur in a latched configuration.
FIG. 3A is a proximal side view of a barrel of the implant of FIG. 1 with a flange extending therethrough.
FIG. 3B is a proximal side view of a barrel with a flange extending therethrough according to another embodiment of the disclosure.
FIG. 3C is a side view of a peg of the fracture fixation system of FIG. 1.
FIG. 4 is a schematic view of a fracture fixation system according to another embodiment of the disclosure implanted into a femur in an unlatched configuration.
FIG. 5 is a schematic view of the fracture fixation system of FIG. 4 implanted into the femur in a latched configuration.
FIG. 6A is a proximal side view of a barrel of the fracture fixation system of FIG. 4 with a flange extending therethrough in an unlatched configuration.
FIG. 6B is a proximal side view of the barrel of the fracture fixation system of FIG. 4 with a flange extending therethrough in a latched configuration.
FIG. 7 is a schematic view of a fracture fixation system according to another embodiment of the disclosure implanted in a femur in a latched configuration secured by an external screw.

### DETAILED DESCRIPTION

The present disclosure describes a fracture fixation system, preferably for a femoral fracture (e.g., femoral neck fractures), which uses the inner lateral cortex of the femur for anchoring of the implant for effective varus control. In consideration of the rotational moments generated on the fracture fixation system by the hip forces applied during typical use of the body, the present invention uses the inner and outer lateral cortices to counteract such rotational moments, as will be described below.

As used herein, the term "proximal," when used in connection with a device or components of a device, refers to the end of the device closer to the user of the device (e.g., surgeon or operator) when the device is being used as intended. On the other hand, the term "distal," when used in connection with a device or components of a device, refers to the end of the device farther away from the user (e.g., surgeon or operator) when the device is being used as intended. As used herein, the term "superior" refers to an upward direction on the page or relative to an anatomy of a person standing upright. On the other hand, the term "inferior" refers to a downward direction on the page or relative to an anatomy of a person standing upright. It should be understood that these terms are not limiting, but merely used for ease of description, and that varied orientations may cause directions to differ. As used herein, the terms "substantially, "generally," "approximately" and "about" are intended to mean that slight deviations from absolute are included within the scope of the term so modified.

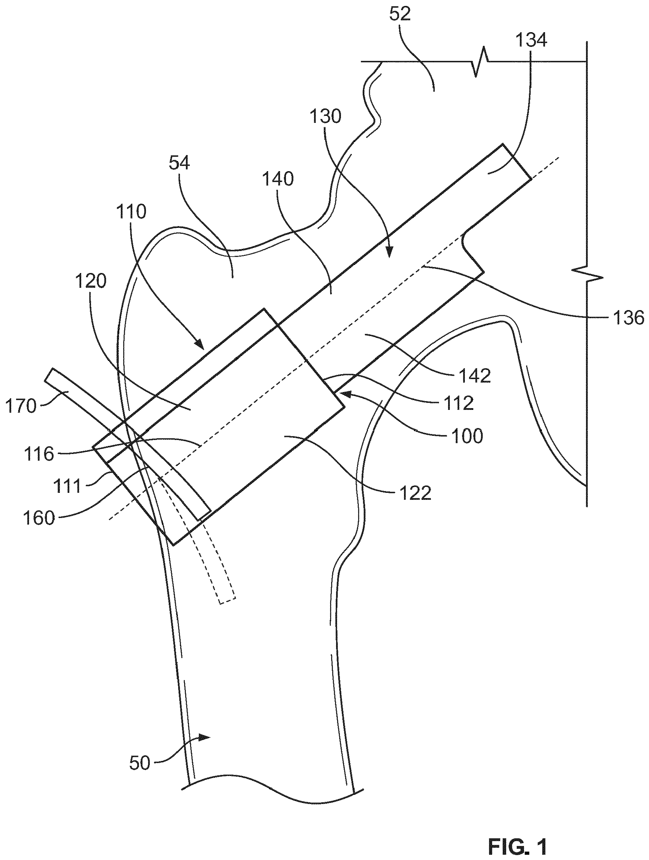

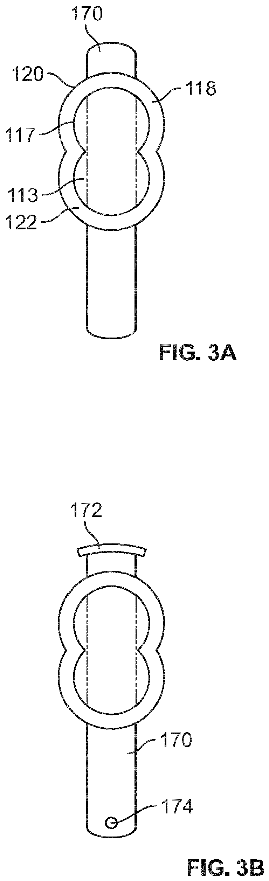

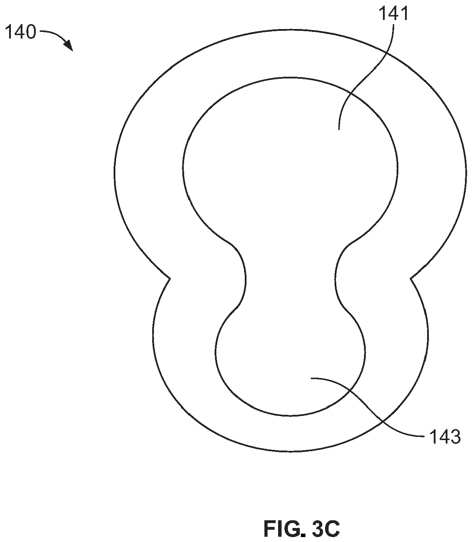

FIGS. 1-3A illustrate a fracture fixation system 100 according an embodiment of the disclosure. Fracture fixation system 100 includes a barrel 110 extending along a barrel axis 116 and having a peripheral wall 118 that extends from a barrel proximal end 111 to a barrel distal end 112 and defines a passage 113 therethrough. Barrel 110 may be manufactured based on patient-specific data to a particular size, shape and profile, which aids in effectively controlling varus forces. Fixation system 100 is designed for use with a proximal femur 50, such that in use barrel 110 extends towards and/or into femoral neck 54. An inner surface 117 of peripheral wall 118 may define a figure-8 shape (shown more clearly in FIG. 3A) that is comprised of overlapped cylindrical surfaces. Barrel 110 includes a first superior cylindrical portion (or surface) 120 and a second inferior cylindrical portion (or surface) 122, the cylindrical surfaces extending parallel to one another. Superior cylindrical surface 120 may have a maximum diameter larger than a maximum diameter of inferior cylindrical surface 122.

Fixation system 100 further includes a peg 130 configured for insertion into passage 113 of barrel 110 and extending along a peg axis 136. The body of peg 130 has an outer surface 134 defining a figure-8 shape in a plane perpendicular to peg axis 136. In a similar manner, inner surface 117 of peripheral wall 118 of barrel 110 has a figure-8 shape in a plane perpendicular to barrel axis 116. The figure-8 shape of outer surface 134 of peg 130 corresponds to and matches the figure-8 shape of inner surface 117 of peripheral wall 118 of barrel 110, so that a non-rotational interlocking fit can be achieved when the peg is disposed within the barrel. That is, the figure-8 shape of outer surface 134 of peg 130 is substantially similar in size and shape or congruent to the figure-8 shape of peripheral wall 118 of barrel 110. This provides an angularly stable and dynamic structure.

Peg 130 is comprised of overlapped cylindrical portions that define the figure-8 shape of its outer surface 134. This includes a first superior cylindrical portion 140 having a maximum diameter and a second inferior cylindrical portion 142 having a maximum diameter. The maximum diameters of superior and inferior cylindrical portions 140, 142 may taper in the proximal-distal direction, *e.g*., as peg 130 extends in the distal direction. Similar to cylindrical portions 120, 122 of barrel 110, the maximum diameter of superior cylindrical portion 140 of peg 130 may be larger than the maximum diameter of inferior cylindrical portion 142. In other embodiments, the maximum diameters of cylindrical portions 140, 142 may be equal or substantially identical. Alternatively, outer surface 134 of peg 130 may be formed by any two non-rotational symmetrical shapes that are overlapped to form a single body. Peg 130 may be monolithic or comprised of multiple components. That is, each cylindrical portion 140, 142 may be a distinct element and joined together for use with barrel 110. With superior cylindrical portion 140 configured to be positioned superior to inferior cylindrical portion 142 in an implanted configuration, an inverted figure-8 shape of peg 130 is achieved, which allows appropriate cut-out resistance while maintaining sufficient post-operative rotational control.

While the described figure-8 shapes are particular to the illustrated embodiment, any non-circular shapes can be used, such as oval, triangular, etc. The non-circular perimeter stabilizes fracture fixation system 100 within the bone to resist rotation of the bone fragments during healing. In some embodiments, the positioned superior and inferior cylindrical portions of the figure-8 shape may be positioned for use with a lag screw in the inferior portion. However, the cylindrical portions can be of the same size or could alternatively be inverted. In other embodiments, a cylindrical shape could be used with two offset holes so that fixation elements can be inserted to create a non-rotational fixation.

Superior and inferior cylindrical portions 140, 142 of peg 130 each define a lumen 141, 143, respectively. In one embodiment, lumen 143 of inferior cylindrical portion 142 has an internal diameter that is larger than an internal diameter of lumen 141 of superior cylindrical portion 140. In an alternative embodiment, the diameters of the lumens may be equal or substantially identical. In use, superior cylindrical portion 140 provides its lumen 141 as a cannulation to allow insertion over a guide wire, for example. Lumen 143 of inferior cylindrical portion 142 offers the option to insert a dedicated instrument such as a screw to actively apply compression, or apposition, intraoperatively.

Superior and inferior cylindrical portions 140, 142 of peg 130 also have different maximum lengths along peg axis 136, wherein the inferior cylindrical portion is shorter than the superior cylindrical portion of the peg. These lengths are measured from the terminal end of the respective cylindrical portion. Both lengths are still longer than a length of barrel 110 which is measured from proximal end 111 of the barrel to the opposite terminal end of the barrel, i.e., distal end 112. In other words, barrel 110 extends from proximal end 111 to distal end 112, and peg 130 extends beyond the distal end of the barrel, with superior cylindrical portion 140 of the peg extending longer than inferior cylindrical portion 142, as illustrated in FIG. 1. The shorter length of inferior cylindrical portion 142 permits insertion of a lag screw through lumen 143 that extends past its distal end. In other embodiments, perhaps in which a lag screw may not be intended for use, the lengths of cylindrical portions 140, 142 may be the same or inverted.

Fixation system 100 may include a mechanism to limit travel of peg 130 within barrel 110, as is further described in U.S. Provisional Patent Application No. 63/310,328 filed February 15, 2022, the disclosure of which is hereby incorporated by reference herein.

Barrel 110 further defines a transverse bore 160 near proximal end 111 of the barrel extending in a direction substantially transverse to barrel axis 116. Transverse bore 160 extends from a superior surface of barrel 110 to an opposing inferior surface of the barrel, through each of superior cylindrical portion 120 and inferior cylindrical portion 122. In this way, transverse bore 160 is completely enclosed within at least two portions of peripheral wall 118. Superior cylindrical portion 120 defines a superior portion of transverse bore 160 and inferior cylindrical portion 122 defines an inferior portion of the transverse bore. In some examples, the superior portion of transverse bore 160 may have a width or diameter larger than a width or diameter of the inferior portion of the transverse bore.

Fixation system 100 further includes a flange 170 sized and shaped to extend through transverse bore 160 as shown in FIGS. 1-3A. Flange 170 extends generally in the superior-inferior direction and may also extend in the proximal-distal direction when in the implanted configuration. Flange 170 is shown in FIG. 1 in a first position, which is a superior or unlatched configuration, wherein an inferior portion of flange 170 extends through transverse bore 160 and a superior portion of flange 170 protrudes or extends generally in the superior direction from barrel 110. That is, in the unlatched configuration, flange 170 may extend from only one of the opposing superior and inferior sides of peripheral wall 118. When flange 170 is preinstalled in the unlatched configuration, fixation system 100 may be implanted in femur 50 so that flange 170 does not interfere with the cortical bone located inferiorly of the implantation site during insertion of the barrel 110 along barrel axis 116.

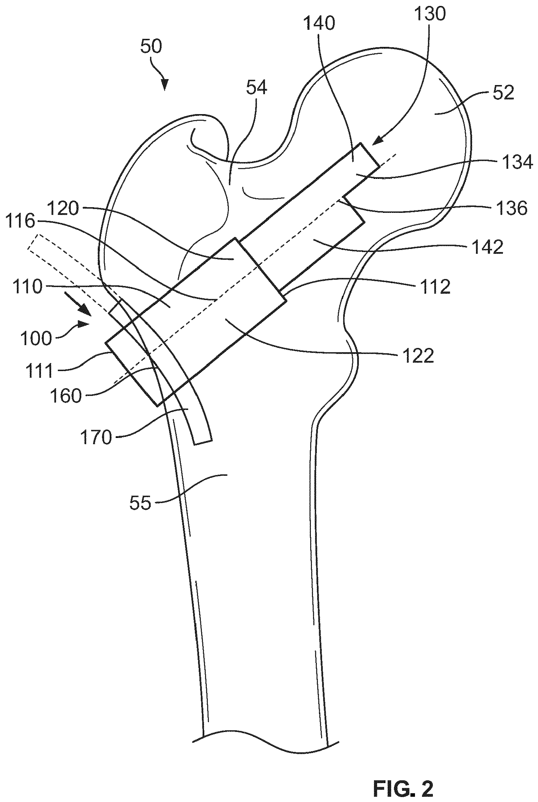

After implantation, flange 170 is configured to be transitioned or moved from the unlatched configuration to a second position, which is an inferior or latched configuration, in which flange 170 is translated generally in the inferior direction through transverse bore 160. The forecasted positioning of flange 170 in the latched configuration is shown in dotted lines in FIG. 1, and the actual positioning of the flange in the latched configuration is shown in FIG. 2. When in the latched configuration, a superior portion of flange 170 is disposed within transverse bore 160 and an inferior portion of flange protrudes or extends from barrel 110 in the inferior direction such that, in the example shown in FIG. 2, flange 170 extends into an intramedullary canal 55 of femur 50 and may contact a lateral cortex of the femur and/or may be positioned in an inner lateral cortex of the femur. Transitioning of flange 170 to the latched configuration may thus secure fixation system 100 against varus forces.

In some examples, a superior end of flange 170 may protrude or extend proximally from femur 50 when fixation system 100 is in the latched configuration, such that flange 170 extends through both of opposing sides of peripheral wall 118. In such examples, the proximally protruding flange 170 may distribute the resulting varus forces generally evenly (e.g., inferior and superior to fixation system 100). In other examples, flange 170 may not protrude or extend from femur 50 at all in the latched configuration, such that the superior end of the flange is fully sunk and/or generally flush with an outer surface of the femur or inserted completely therein. When in the latched configuration, flange 170 may be adjusted in the proximal-distal direction to be properly seated within femur 50. In the examples in which the superior portion of transverse bore 160 has a larger width or diameter than the inferior portion, flange 170 may have a larger space to be adjusted nearer the superior end (e.g., the portion of the flange disposed in the superior portion of the transverse bore) relative to the inferior end of the flange (e.g., the portion of the flange disposed in the inferior portion of the transverse bore).

In some examples, flange 170 may include a superior stop 172 and an inferior stop 174 to maintain the flange within transverse bore 160 of barrel 110 as shown in FIG. 3B. Superior stop 172 may be a shoulder, protrusion, or an extended width portion formed monolithically with the flange having a width greater than the width of flange or thereby increasing the width of the flange. Superior stop 172 may alternatively be a separate piece coupled to flange 170 such as a pin, screw, peg or the like. The width (*e.g*., the dimension measured in the proximal-distal direction) of superior stop 172 is also larger than a width or diameter of transverse bore 160 so that the superior stop is configured to abut a surface of superior cylindrical portion 120 and thereby prevented from passing through the transverse bore when the flange is moved in the inferior direction. Superior stop 172 may also or alternatively have a depth (*e.g*., the dimension measured in a direction perpendicular to the width) that is larger than a depth or diameter of transverse bore 160 to prevent the superior stop from passing through the transverse bore. Inferior stop 174 may similarly be a shoulder, protrusion, extended width portion formed monolithically with flange 170 or alternatively a pin, screw or peg coupled to the flange and extending adjacent a distal end of the flange. In some examples, inferior stop 174 may be substantially similar to superior stop 172. Alternatively, inferior stop 174 may extend from flange 170 such that the combined width of the flange and inferior stop is greater than the width or diameter of transverse bore 160 as shown in FIG. 3B, and the inferior stop is configured to abut a surface of inferior cylindrical portion 122 of barrel 110 and thereby prevented from passing through the transverse bore when the flange is moved in the superior direction. It is contemplated that fixation system 100 may be pre-assembled prior to being implanted into femur 50, particularly in examples in which flange 170 includes superior and inferior stops 172, 174.

In certain examples including an inferior stop 174, the inferior stop may be assembled to flange 170 while the flange is inserted into transverse bore 160 such that an inferior portion of the flange is positioned inferior to superior cylindrical portion 140, *i.e.,* positioned within inferior cylindrical bore 142 or below. That is, inferior cylindrical portion 142 may define a recess or opening sized to permit movement of inferior stop 174 therethrough in the superior-inferior direction, whereas superior cylindrical portion 140 does not define such a recess and inferior stop 174 is prevented from passing through the superior cylindrical portion. As such, after assembly of inferior stop 174 to flange 170, the flange is still permitted to be translated in the superior-inferior direction, but an inferior portion of the flange (*i.e.,* the portion on which the inferior stop is positioned on the flange) is inhibited from moving superiorly beyond inferior cylindrical portion 142. In other words, a superior portion of flange (*i.e.,* the portion of flange above inferior stop 174) may be permitted to pass through superior cylindrical portion 140, but an inferior portion of flange (*i.e.,* the portion of flange at and below inferior stop 172) may only be permitted to pass through inferior cylindrical portion 142 and below.

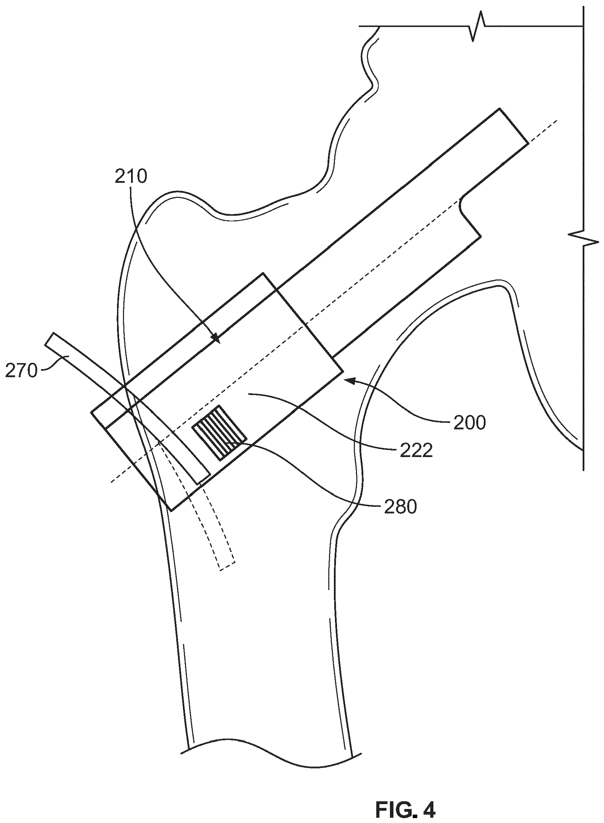

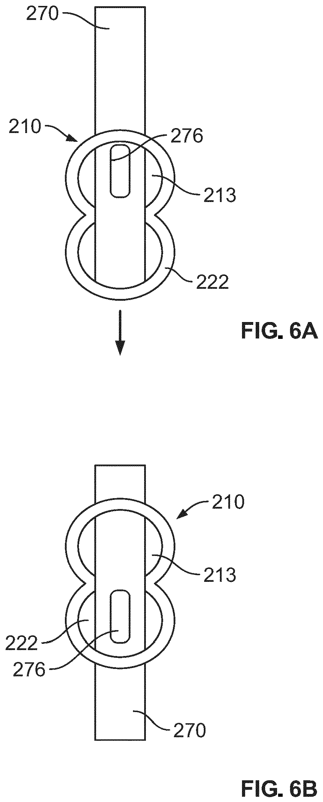

FIGS. 4-6B illustrate a fixation system 200 according to another embodiment of the disclosure. Fixation system 200 is substantially similar to fixation system 100, and thus like reference numerals are used for like elements of fixation system 200, but in the 200-series of numbers. Fixation system 200 includes all components identified above with reference to fixation system 100, with the addition of an internal screw 280. Internal screw 280 is coupled to and disposed within barrel 210, preferably in or near inferior cylindrical portion 222. An additional distinction is that fixation system 200 includes flange 270 defining an opening 276 extending therethrough as shown in FIGS. 6A-6B. Opening 276 of flange 270 is configured to align with internal screw 280 when the flange is in an inferior position or the latched configuration. Internal screw 280 may be accessible for actuation by a user through passage 213 of barrel 210 and through opening 276 of flange 270 when the flange is in or near the latched configuration. Opening 276 may be oblong in shape and may be substantially similar in size and shape to internal screw 280 so that the screw is accessible. Opening 276 may be slightly smaller or larger than internal screw 280. Internal screw 280 may be actuated by a user to be translated in a proximal direction toward flange 270 to abut a distal face of the flange and additionally apply pressure to the flange, pushing an inferior portion of the flange into closer (*e.g*., substantially close) contact or full contact with the inner lateral cortex of femur 50. After being actuated, the pressure of internal screw 280 on the distal portion of flange 270 may also cause flange 270 to pivot about its middle portion such that a superior portion of flange 270 is pressed toward and/or against the outer lateral cortex of femur 50. The pressure points created by actuation of internal screw 280 (*e.g*., the inferior portion of the flange against the inner lateral cortex and the superior portion of the flange against the outer lateral cortex) may increase the stability of flange 270 and fixation system 200 overall.

In some examples, opening 276 of flange 270 may be configured to receive at least a portion of internal screw 280 when the flange is in an inferior position or in the latched configuration. Actuation of internal screw 280 may translate the internal screw through opening 276 of flange 270, either with or without applying pressure against the flange as described above (depending on the size and shape of the internal screw and the opening of the flange). By positioning internal screw 280 to extend through opening 276 of flange 270, the flange may be prevented from moving in the superior direction and thereby locked in the latched configuration to enhance the stability of fixation system 200.

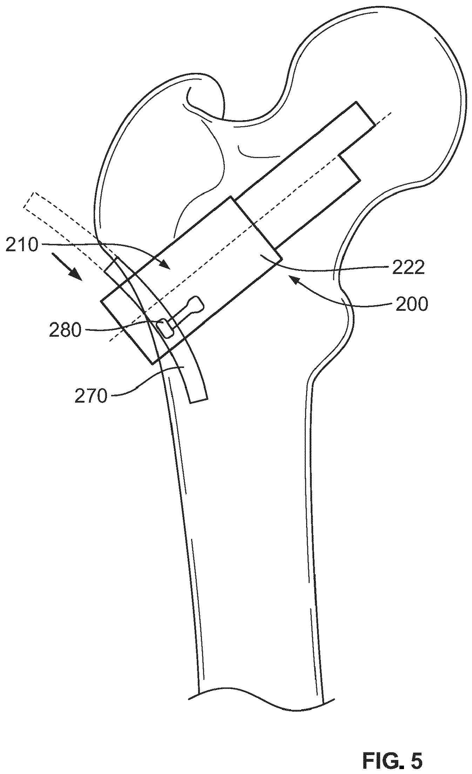

Similar to fixation system 100 described above, fixation system 200 is shown in the unlatched configuration in FIG. 4 with flange 270 extending in a superior direction from barrel 210. In FIG. 5, fixation system 200 is shown in a latched and locked configuration. That is, flange 270 is moved in the inferior direction aligning opening 276 with internal screw 280, and the internal screw is actuated to (1) apply pressure against the inferior portion of the flange, (2) be passed through the opening to lock the flange in the latched configuration, or both apply pressure and pass through to lock the flange. The alignment of opening 276 with respect to barrel 210 is illustrated more clearly in FIGS. 6A-6B. FIG. 6A shows flange in the unlatched configuration, whereas FIG. 6B shows the flange in the latched configuration, wherein opening 276 is generally aligned with inferior cylindrical portion 222 of barrel 210. It is contemplated that internal screw 280 and opening 276 may be positioned anywhere on barrel 210 and flange 270, respectively. For example, the internal screw may be positioned in the superior cylindrical portion of the barrel, and the opening may be positioned more proximally or further in the superior direction along the flange relative to the opening in the example shown in FIGS. 4-6B.

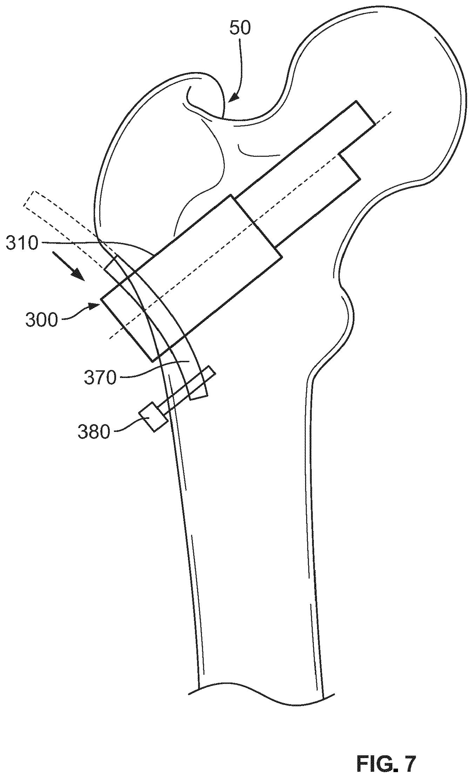

In other examples, flange 370 may be locked in the latched configuration by an external screw 380 as shown in FIG. 7. Fixation system 300 is substantially similar to fixation system 200, and thus like reference numerals are used for like elements of fixation system 200, but in the 300-series of numbers. Flange 370 may define an opening substantially similar to flange 270, and external screw 380 may be configured to be inserted directly into femur 50 along a path aligned with the opening of the flange while the flange is in the latched configuration. External screw 380 may be used for a substantially similar purpose as internal screw 280 described above. That is, external screw 380 may be inserted through the opening of flange 370 to apply a proximal pressure to an inferior portion of the flange, possibly pulling the inferior portion of the flange into closer (*e.g*., substantially close) contact or full contact with the inner lateral cortex of femur 50. For example, the opening of flange 370 may have an internal threading that engages with an external threading of external screw 380 to draw the flange proximally toward the inner lateral cortex. The pressure of external screw 380 on the inferior portion of flange 370 may also cause the flange to pivot about its middle portion such that a superior portion of the flange is pressed toward and/or against the outer lateral cortex of femur 50. The pressure points created by actuation of external screw 380 (*e.g*., the inferior portion of the flange against the inner lateral cortex and the superior portion of the flange against the outer lateral cortex) may increase the stability of flange 370 and fixation system 300 overall. Use of external screw 380 may help achieve rotational control of fixation system 300 in the coronal plane. Fixation system 300 and external screw 380 may be separate elements and may be delivered in a kit.

A method of using fracture fixation system 100 described above may include a preliminary step of inserting a k-wire through femoral neck 54 and into femoral head 52. While this step is not required, it is useful to provide alignment for the following drilling and insertion steps. Next, superior and inferior bore holes are drilled through femoral neck 54 and toward/into femoral head 52, such that the bore holes at least partially overlap to create a bore hole having a figure-8 shape. This shape can match any desired silhouette or outline of a barrel as described above, such that the figure-8 shape of the bore hole and the figure-8 shape of peripheral wall 118 of barrel 110 to be used are substantially similar in size and shape. For example, the inferior bore hole can be of a smaller diameter than the superior bore hole. Assuming a k-wire is used, at least the superior bore is drilled over the k-wire using a cannulated drill bit.

Fracture fixation system 100 is mounted to femur 50, including inserting peripheral wall 118 of barrel 110 into the bore hole. Peg 130 is inserted into a passage defined through barrel 110 of fracture fixation system 100 such that a distal end of the peg extends into communication with femoral head 52. Again, assuming the k-wire is used, these steps can include guiding the passage of fracture fixation system 100 over k-wire and guiding lumen 141 of superior cylindrical portion 140 over the k-wire. Peg 130 can be preloaded into fixation barrel 110 without the need for any tooling, so that the steps for inserting the barrel and the peg are carried out together, for example with a targeting device or other insertion instrument.

In some embodiments, a threaded lag screw may be inserted into lumen 143 of inferior cylindrical portion 142 of peg 130 and into femoral head 52.

In an alternative method to that described above, either before or after the superior and inferior bore holes are drilled, peg 130 can be assembled with barrel 110 prior to insertion of either component. That is, peg 130 may be inserted into the passage defined by barrel 110 while outside of the bone. Then, peg 130 and barrel 110 may together be mounted to the bone by inserting the barrel and at least a portion of the peg into the bore hole to the position described above.

Each of the method steps described above may be completed with flange 170 in a superior position or unlatched configuration. After barrel 110 and peg 130 are substantially or fully inserted into the proximal portion of femur 50 such that, for example, the peg extends into femoral head 52, flange 170 may be moved in the inferior direction through transverse bore 160 of the barrel to transition the flange from the superior position or unlatched configuration to the inferior position or latched configuration. Flange 170 may be transitioned by, for example, being pressed or pushed down by the hand of a surgeon or operator or with a tool such as calipers, a mallet or the like. After being transitioned to the latched configuration, flange 170 may be adjusted in the proximal-distal direction (or still further in the superior-inferior direction) to ensure that the flange is desirably seated within femur 50. In some examples, such as fixation system 200, after flange 270 has been moved to the latched configuration, opening 276 of the flange may be aligned with internal screw 280, and the internal screw may be accessed through passage 213 of barrel 210 to be actuated and passed through opening 276 of the flange to thereby lock the flange in the latched configuration. In other examples, such as fixation system 300, after flange 370 has been transitioned to the latched configuration, an external screw 380 may be inserted into femur 50 and through the opening of flange 370 to lock the flange in the latched configuration. External screw 380 may also contact flange 370 and translate flange toward the lateral cortex of femur 50. The positioning of opening 380 may be known relative to barrel 310. That is, when flange 370 is in the latched configuration, a surgeon may be able to find an insertion point and angle for external screw 380 through femur 50 to be aligned with the opening of the flange by measuring from barrel 310. Alternatively, the length of flange 370 may be known and the location of the opening may be found by measuring from a superior/proximal or exposed portion of the flange.

While the embodiments and methods have been described in connection with a femur, use of the present embodiments with a humerus, a tibia, or any other bone is contemplated.

Each component of fracture fixation system 100 may be formed by an additive manufacturing process, including but not limited to electron beam melting (EBM), selective laser sintering (SLS), selective laser melting (SLM), binder jet printing, and blown powder fusion for use with metal powders. This is particularly beneficial as the silhouette of the figure-8 shape or other non-circular shape can be made with a specific patient's anatomy in mind, specifically to narrow, widen, lengthen, and/or shorten any of the dimensions of fracture fixation system 100. Each component of fracture fixation system 100 can be made of any surgical grade material, and particularly various metals such as titanium, titanium alloys, stainless steel, cobalt chrome alloys, tantalum and niobium, or any combination thereof. Gold and/or silver can be provided in the material composition or as a coating of a component. It is contemplated that certain components may be formed of or coated in various metal ions having anti-microbial properties.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A fracture fixation system (100), comprising:
a barrel (110) extending along a barrel axis (116) from a proximal end (111) to a distal end (112) and having a wall (118), the barrel (110) defining a passage (113) through the barrel (110) that extends along the barrel axis (116), wherein the wall (118) defines a transverse bore (160) passing through opposing sides of the wall (118) at the proximal end (111) of the barrel (110) in a direction that is substantially transverse to the barrel axis (116);
a peg (130) extending along a peg axis (136) and configured for insertion into the passage (160); and
a flange (170) configured for insertion into the transverse bore (160) of the wall (118).

2. The fracture fixation system of claim 1, wherein the flange (170) is moveable in opposing directions through the transverse bore (160) of the barrel (110).

3. The fracture fixation system of any one of claims 1 or 2, wherein in a pre-implanted configuration, the flange (170) extends from one of the opposing sides of the wall (118).

4. The fracture fixation system of claim 3, wherein in the pre-implanted configuration, the flange (170) extends in a superior direction from the barrel (110).

5. The fracture fixation system of any one of claims 1-4, wherein in an implanted configuration, the flange (170) extends through both of the opposing sides of the wall (118).

6. The fracture fixation system of claim 5, wherein in the implanted configuration, the flange (170) contacts a lateral cortex of the femur (50).

7. The fracture fixation system of any of claim 5, wherein in the implanted configuration, a proximal end of the flange (170) is flush with the barrel (110).

8. The fracture fixation system of claim 5, wherein in the implanted configuration with the flange (170) in a latched configuration, the flange (170) protrudes in a superior direction from the barrel (110).

9. The fracture fixation system of claim 5, wherein in the implanted configuration, a distal portion of the flange (170) abuts an inner lateral cortex of a femur (50).

10. The fracture fixation system of any one of claims 1-9, further comprising a screw (280) configured to be accessible through an oblong opening (276) defined in the flange (270).

11. The fracture fixation system of any one of claims 1-10, wherein the barrel (110) includes a superior cylindrical portion (120) defining a superior portion of the transverse bore (160) and the barrel (110) includes an inferior cylindrical portion (122) defining an inferior portion of the transverse bore (160).

12. The fracture fixation system of claim 11, wherein the inferior portion of the transverse bore (160) has a diameter larger than a diameter of the superior portion of the transverse bore (160).

13. The fracture fixation system of any one of claims 1-12, wherein an inner surface of the wall (118) of the barrel (110) has a non-circular shape in a plane perpendicular to the barrel axis (116), and a body of the peg (130) has an outer surface defining a non-circular shape in a plane perpendicular to the peg axis (136).

14. The fracture fixation system of claim 13, wherein the non-circular shapes of the inner surface of the wall (118) of the barrel (110) and of the outer surface of the body of the peg (130) are figure-8 shapes.

15. A kit comprising:
the fracture fixation system (200) of any one of claims 1-14;
a screw (280) for insertion through a distal portion of the flange (270).
